# EUROPEAN PATENT SPECIFICATION

(11) **EP 0 535 390 B1**
(45) Date of publication and mention of the grant of the patent: **15.05.1996**
(21) Application number: 92115046.2
(22) Date of filing: 03.09.1992
(51) Int. Cl.: A61K 31/425

(54) **The use of L-2-oxothiazolidine-4-carboxylate for the preparation of a medicament for treating latent HIV infection**
Verwendung von L-2-Oxothiazolidine-4-Carboxylate zur Herstellung eines Arzneimittels zur Behandlung von latenten HIV-Infektionen
Utilisation de L-2-oxothiazolidine-4-carboxylate pour la fabrication d'un médicament pour le traitement d'infection latente de HIV

(30) Priority: 30.09.1991 US 769194
(43) Date of publication of application: 07.04.1993
(73) Proprietor: Transcend Therapeutics, Inc., Cambridge Massachusetts 02139 (US)
(72) Inventor: Goldberg, Dennis I., Hawthorn Woods, IL 60047 (US); Mark, David A., Oak Park, Illinois 60304 (US); Rowe, W. Bruce, Evanston, Il 60202 (US); Thompson, Kathleen A., Palatine, IL 60067 (US)
(74) Representative: MacGregor, Gordon

(56) References cited:
- EP-A- 0 057 942
- PROC. NATL. ACAD. SCI. USA vol. 88, February 1991, pages 986 - 990; T. KALEBIC ET AL.
- PROC. NATL. ACAD. SCI. USA vol. 87, December 1990, pages 9943 - 9947; F.J.T. STAAL ET AL.
- ACTA VIROL. vol. 11, no. 6, 1967, pages 559 - 561; V. FRANKOVA.
- PHOTOCHEMISTRY AND PHOTOBIOLOGY vol. 53, no. 1, January 1991, pages 85 - 92; D. K. GAFFNEY ET AL. '
- CURRENT THERAPEUTIC RESEARCH vol. 52, no. 3, September 1992, pages 461 - 467; G.GIORGI ET AL.
- Dorland's Illustrated Medical Dictionary, 27th ed., p. 1207 and 1725
- Biochemistry, The Chemical Reactions of Living Cells, David E. Metzler, 1977, Academic Press, Inc., pages 814-815
- Biochemistry, 2nd ed., Alber L. Lehninger, Worth Publishers, Inc. pages 794-796
- Biochemical Pharmacology, vol. 39(12), 1990, pages 1877-1881.

## Description

### BACKGROUND OF THE INVENTION

The present invention relates to the stimulation of the intracellular synthesis of glutathione for the treatment of latent HIV infection.

Acute viral infections result from exposure of the host to an infectious viral agent. The nature and clinical symptoms associated with the infection will vary depending upon the virus present. In many non-lethal acute viral infections, the host organism will mount an effective immune response to the invading virus and eventually clear the virus from its system entirely. Other viruses establish chronic infections in which viral replication and associated symptomatology occur continuously throughout the life of the host organism.

A smaller number of viruses, however, establish a different life cycle pattern. Infections caused by these viruses are marked by an initial, occasionally asymptomatic infection during which viral replication occurs. This is followed by a period in which infectious viral particles are not produced. The viral genome remains within the cells of its host organism but does not replicate. This period of persistent, but non-active, infection has been termed latent viral infection.

It appears probable that HIV-1, the virus responsible for Acquired Immunodeficiency Syndrome (AIDS) in humans, undergoes a period of latent activity. Schnittman, et al, The Reservoir for HIV-1 in Human Peripheral Blood is a T Cell That Maintains Expression of CD4, Science, Vol. 245, pp. 305-308, 1989.

Of clinical significance is the finding that this latent virus can, upon appropriate stimulation, reactivate. Reactivation gives rise to both the production of infectious viral particles and the appearance of symptomatology associated with recurrent infection. A variety of stimuli have been reported to reactivate latent viruses, including (but not limited to): fever; local trauma; exposure to sunlight or exogenous chemicals (including some medications); trigeminal nerve manipulation; menstruation; malnutrition; physical or emotional stress; concurrent infections with other pathogens; and alterations in immune status.

Although the conditions that will precipitate viral reactivation have been identified, the understanding of the mechanism(s) by which these conditions reactive viral gene transcription is very limited. HIV-1 appears to be the most thoroughly studied example in this regard.

HIV-1 viral gene transcription occurs at a very low or negligible level in unstimulated monocyte or lymphocyte cell lines despite the presence of an integrated HIV proviral genome. Exposure of these cells to a number of different agents (including various cytokines) stimulates viral gene transcription and translation. The mechanism of this stimulation has been shown to involve the activation via a phosphorylation reaction of a cellular (i.e., host) transcription factor designated NF-κB which is released from an inhibitory protein complex in the cytoplasm and translocates to a cell nucleus. Duh, et al, Tumor Necrosis Factor α Activates Human Immunodeficiency Virus Type 1 Through Induction of Nuclear Factor Binding to the NF-κB Sites in the Long Terminal Repeat, Proc. Nat. Acad. Sci. Vol. 86, pp. 5974-5978, 1989.

A variety of cellular genes encoding proteins involved in immune responses have been reported to contain NF-κB binding sites. In a classic example of viral exploitation of host cell physiology, the proviral HIV-1 genome also contains NF-κB binding sites. Binding of NF-κB to the HIV-1 promoter region of the proviral genome "turns on" viral gene transcription, allowing the virus to escape latency and reactivating the viral infection.

NF-κB-mediated enhancement of HIV-1 replication may be effected by the intracellular thiol concentration. It is reported that intracellular thiol levels regulate the activation of NF-κB levels. Staal, et al, Intracellular Thiols Regulate Activation of Nuclear Factor κB and Transcription of Human Immunodeficiency Virus, Proc. Nat. Acad. Sci., Vol. 87, pp. 9943-9947, 1990. N-acetyl-L-cysteine blocks NF-κB activation, and HIV transcription in vitro. See, Staal et al, id.

The addition of the thiol-containing compounds glutathione, glutathione ester can also suppress the induction of HIV-1 expression. (Kalebic, et al, Suppression of Human Immunodeficiency Virus Expression in Chronically Infected Monocytic Cells by Glutathione, Glutathione Ester, and N-acetylcysteine, Proc. Nat. Acad. Sci. Vol. 88, pp. 986-990, 1991).

It has also been reported that cysteine can inhibit HIV-1 replication in persistently infected cells. "However, inhibition of HIV-1 replication appears not to be directly correlated with GSH levels." (Mihm, et al, Inhibition of HIV-1 Replication and NF-κB Activity by Cysteine and Cysteine Derivatives, AIDS, Vol. 5, pp. 497-503, 1991).

### SUMMARY OF THE INVENTION

The present invention provides a therapy for the treatment of latent HIV infection. Specifically, the present invention provides the use of L-2-oxothiazolidine-4-carboxylate (OTC) for the preparation of a medicament that can be used for both the prophylactic and active treatment of latent HIV infections by increasing the intracellular synthesis of cysteine and/or glutathione.

By maintaining plasma and tissue glutathione levels, the present invention prevents the symptomatology associated with latent HIV infections. Additionally, to the extent the virus is reactivated, the method of the present invention facilitates a return to latency.

In an embodiment of the present invention, the medicament is designed to be administered enterally.

In an embodiment of the present invention, the medicament is designed to be administered parenterally.

In an embodiment of the present invention, the medicament is designed to be administered during the latent phase of the viral infection.

In an embodiment of the present invention, the medicament is designed to be administered during an active phase of the viral infection and accelerates a return of the viral infection to the latent stage.

Additional features and advantages of the present invention are described in, and will be apparent from, the detailed description of the presently preferred embodiments.

### DETAILED DESCRIPTION OF THE PRESENTLY PREFERRED EMBODIMENTS

The present invention provides use of L-2-oxothiazolidine-4-carboxylate for the manufacture of a medicament for treating latent HIV infection and for maintaining such infection in a latent state and hastening the return to latency after a recurrent viral reactivation.

A number of conditions associated with reactivation of the latent viruses are believed by the inventors to be associated with decreased levels of glutathione. These factors include trauma and stress conditions involving inflammatory reactions, malnutrition, and exposure to sunlight. These factors create periods of oxidative stress. The inventors believe that the cellular thiol status may play an important role in triggering the onset and influencing the duration of active infection.

Pursuant to the present invention, the medicament increases the intracellular glutathione levels of cells harboring latent or actively expressed "latent" virus.

Pursuant to the present invention, a non-cysteine substrate that is a precursor for the intracellular synthesis of glutathione is administered to the patient. Because the substrate is not a cysteine substrate, a more effective tissue distribution of the substrate occurs within the patient without the potentially toxic elevations of plasma cysteine levels associated with direct administration of the amino acid cysteine.

It is believed that the enzymes necessary for deacetylization of an acetylated compound such as n-acetylcysteine exist only in the kidney. Accordingly, a compound such as n-acetylcysteine must be metabolized to cysteine in the kidney then transported to the liver or peripheral cells. Therefore, such compounds may not be sufficiently distributed to the requisite tissues of the patient, i.e., the cells harboring the latent or activated virus.

Pursuant to the present invention, L-2-oxothiazolidine-4-carboxylate is used. L-2-oxothiazolidine-4-carboxylate, in vitro, is subjected to the action of 5-oxo-L-prolinase in the presence of adenosine triphosphate to produce S-carboxyl cysteine. S-carboxyl cysteine is then decarboxylated to produce cysteine. Cysteine can then be further metabolized to produce glutathione. See, U.S. Patent Nos.: 4,335,210; 4,434,158; 4,438,124; 4,665,082; and 4,647,571, the disclosures of which are incorporated herein by reference.

L-cysteine is the rate limiting substrate in the synthesis of cellular glutathione. The κₘ for γ-glutamylcysteine synthetase is equivalent to the normal intracellular cysteine concentration. An increase in the demand for glutathione will deplete intracellular cysteine unless an alternative source, such as L-2-oxothiazolidine-4-carboxylate is present.

In an embodiment of the invention, the composition of the present invention includes: 3% L-2-oxothiazolidine-4-carboxylate in a phosphate buffer. Additional embodiments include:
a) A buffered (pH 6.5 - 7.5) 3% or 6% L-2-oxothiazolidine-4-carboxylate aqueous solution.
b) A buffered 3% or 6% L-2-oxothiazolidine-4-carboxylate aqueous solution containing any of the following alone or in appropriate combinations: amino acids, dextrose or other carbohydrate sources, and lipid emulsions.
c) A vial containing OTC in crystalline or lyophilized form to which appropriate aqueous solutions are added at time of use.
d) A gelatin capsule containing crystalline or lyophilized OTC.
e) A pill containing crystalline or lyophilized OTC.
f) A liquid elemental, protein hydrolysate, carbohydrate and/or lipid emulsion containing enteral dietary supplement containing OTC.

The composition of the present invention can be administered to a patient having latent HIV infection, prophylactically, during the latent stage of the infection.

The composition of the present invention can also be administered to a patient during the active phase of the infection to accelerate a return of the latent stage.

The composition can either be administered alone or as an adjunct therapy with other typical therapies. For example, antiviral agents, interferon, steroids, analgesics, non-steroid anti-inflammatories, and antibiotics can be administered.

By way of example, but not limitation, a contemplated example of the present invention will now be given.

### EXAMPLE

A 39-year old male homosexual with a history of several sexually transmitted diseases was found to be HIV-1 seropositive. The patient's lifestyle indicated that previous exposure to cytomegalovirus was probable. This was confirmed with the demonstration of the presence of serum antibodies to cytomegalovirus, indicating that the patient was latently infected with the virus.

Given the increased morbidity associated with cytomegalovirus reactivation in this patient group, a chronic administration of L-2-oxo-thiazolidine-4-carboxylate was prescribed (500 mg capsule, t.i.d). Although the patient contracted a variety of opportunistic bacterial infections over the five year course of this chronic therapy, the patient never demonstrated any symptoms of cytomegalovirus-associated bowel disease (despite a high predictive risk of developing this ailment).

## Claims

1. Use of L-2-oxothiazolidine-4-carboxylate for the preparation of a composition for treating a patient having latent HIV infection.

2. Use of L-2-oxothiazolidine-4-carboxylate for the preparation of a composition for the prophylactic prevention of the reactivation of latent HIV infection during a period of latency in a patient having latent HIV infection.

3. The use according to Claim 1 or 2 wherein the composition is administrable parenterally.

4. The use according to Claim 1 or 2 wherein the composition is administrable enterally.

5. The use according to Claim 1, 3, or 4 for treating a patient who has exhibited symptoms demonstrating a reactivation of the HIV virus.

6. The use according to Claim 1, 3, or 4 for treating a patient during a latent stage of the HIV infection.

## Patentansprüche

1. Verwendung von L-2-Oxothiazolidin-4-Carboxylat für die Herstellung einer Zusammensetzung zum Behandeln eines Patienten, der eine latente HIV-Infektion aufweist.

2. Verwendung von L-2-Oxothiazolidin-4-Carboxylat für die Herstellung einer Zusammensetzung für die prophylaktische Verhinderung der Reaktivierung der latenten HIV-Infektion während einer Latenzperiode bei einem Patienten mit einer latenten HIV-Infektion.

3. Verwendung nach Anspruch 1 oder 1, wobei die Zusammensetzung parenteral verabreichbar ist.

4. Verwendung nach Anspruch 1 oder 2, wobei die Zusammensetzung enteral verabreichbar ist.

5. Verwendung nach Anspruch 1, 3 oder 4 zum Behandeln eines Patienten, der sichtbare Symptome aufweist, welche eine Reaktivierung des HIV-Virus zeigen.

6. Verwendung nach Anspruch 1, 3 oder 4 zum Behandeln eines Patienten während eines latenten Zustandes der HIV-Infektion.

## Revendications

1. Utilisation du L-2-oxothiazolidine-4-carboxylate pour la préparation d'une composition pour le traitement d'un patient ayant une contamination latente par HIV.

2. Utilisation de L-2-oxothiazolidine-4-carboxylate pour la préparation d'une composition pour la prévention prophylactique de la réactivation d'une infection latente par HIV pendant une période de latence chez un patient ayant une infection latente par HIV.

3. Utilisation selon la revendication 1 ou 2 où la composition peut être administrée par voie parentérale.

4. Utilisation selon la revendication 1 ou 2 où la composition peut être administrée par voie entérale.

5. Utilisation selon la revendication 1, 3 ou 4 pour le traitement d'un patient qui a présenté des symptômes démontrant une réactivation du virus HIV.

6. Utilisation selon la revendication 1, 3 ou 4 pour traiter un patient pendant un stade latent d'une infection par HIV.
